(19) Europäisches Patentamt
European Patent Office
Office européen des brevets

(11) Veröffentlichungsnummer: **0 057 362**
**B1**

(12) # EUROPÄISCHE PATENTSCHRIFT

(45) Veröffentlichungstag der Patentschrift: **20.06.84**

(51) Int. Cl.³: **C 07 D 307/91**, C 07 D 453/02, C 07 D 487/04, A 01 N 43/00

(21) Anmeldenummer: **82100301.9**

(22) Anmeldetag: **18.01.82**

(54) **Dibenzofuranderivate, Verfahren zu ihrer Herstellung und ihre Verwendung zur Bekämpfung von Pilzen.**

(30) Priorität: **30.01.81 DE 3103069**

(43) Veröffentlichungstag der Anmeldung:
**11.08.82 Patentblatt 82/32**

(45) Bekanntmachung des Hinweises auf die Patenterteilung:
**20.06.84 Patentblatt 84/25**

(84) Benannte Vertragsstaaten:
**AT BE CH DE FR GB IT LI LU NL SE**

(56) Entgegenhaltungen:
**EP - A - 0 017 209**
**CH - A - 606 291**
**DE - A - 2 149 923**
**US - A - 3 929 802**

**CHEM. WEEK, 111, 39 (1972)**
**PEST. SCI., 10, 393 (1979)**

(73) Patentinhaber: **BASF Aktiengesellschaft,**
**Carl-Bosch-Strasse 38, D-6700 Ludwigshafen (DE)**

(72) Erfinder: **Rentzea, Costin, Dr., Neuenheimer Landstrasse 72, D-6900 Heidelberg (DE)**
Erfinder: **Feuerherd, Karl-Heinz, Dr., c/o BASF Japan Ltd C.P.O. Box 1757, Tokyo 100-91 (JP)**
Erfinder: **Zeeh, Bernd, Dr., Thorwaldsenstrasse 5, D-6700 Ludwigshafen (DE)**
Erfinder: **Sauter, Hubert, Dr., Neckarpromenade, D-6800 Mannheim (DE)**
Erfinder: **Pommer, Ernst-Heinrich, Dr., Berliner Platz 7, D-6703 Limburgerhof (DE)**

**EP 0 057 362 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Dibenzofuranderivate, Verfahren zu ihrer Herstellung und fungizide Mittel, die diese Verbindungen als Wirkstoffe enthalten, sowie ein Verfahren zur Bekämpfung von Pilzen mit diesen Wirkstoffen.

Es ist bekannt, Tetramethylthiuram-disulfid als Fungizid zu verwenden (Chem. Week, 111, 39 (1972)). Bereits bekannt sind auch andere Fungizide, beispielsweise das 2-Aminobenzimidazolcarbamat (R. Wegler, «Chemie der Pflanzenschutz- und Schädlingsbekämpfungsmittel» Bd. 4, Seite 180, Springer Verlag Berlin/Heidelberg/New York, 1977) und das 1-(Isopropylcarbamoyl)-3-(3,5-dichlorphenyl)-imidazolidin-2,4-dion (DE-OS 21 49 923), die bei phytopathogenen Pilzen, wie zum Beispiel Botrytis cinerea, mindestens genau so stark wirken wie das Tetramethylthiuram-disulfid. 2-Aminobenzimidazol-carbamat erzeugt jedoch schnell Resistenz bei einer sehr grossen Zahl von phytopathogenen Pilzen (loc. cit., Seite 92 bis 93 und 171 bis 172) und 1-(Isopropylcarbamoyl)-3-(3,5-dichlorphenyl)-imidazolidin-2,4-dion lagert sich spontan zum inaktiven 1-(3,5-Dichlorphenylcarbamoyl)-3-isopropyl-imidazolidin-2,4-dion um (Pestic. Sci., 10, 393 (1979)).

Es wurde gefunden, dass Dibenzofuranderivate der Formel

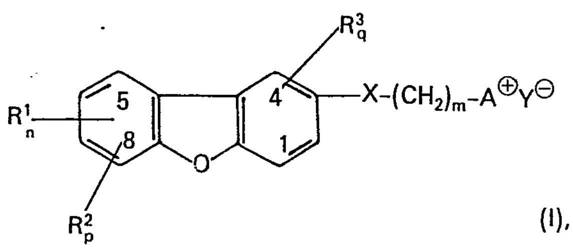

(I),

in der

$R^1$, $R^2$ und $R^3$ gleich oder verschieden sind und Halogen, eine gegebenenfalls halogensubstituierte Alkyl- oder Alkoxygruppe mit 1 bis 4 Kohlenstoffatomen, Cyan oder Nitro,

n, p und q die Zahlen 0, 1, 2 oder 3,

X Sauerstoff oder Schwefel,

m die Zahlen 2, 3 oder 4,

A einen Chinuclidin- oder Pyrrolizidinbicyclus oder die Gruppe $-N^{\oplus}R^4R^5R^6$,

in der $R^4$, $R^5$ und $R^6$ gleich oder verschieden sind und unabhängig voneinander für Alkylreste mit bis zu 6 Kohlenstoffatomen für Allyl, 2-Butenyl, 4-Chlor-2-butenyl, Propargyl, für Cycloalkylreste mit bis zu 12 Kohlenstoffatomen oder Cycloalkenylreste mit bis zu 7 Kohlenstoffatomen, wobei diese acyclischen und cyclischen Reste durch Halogen, Cyan, Alkoxy mit 1 bis 4 Kohlenstoffatomen oder Dialkylamino mit 1 bis 4 Kohlenstoffatomen in einer Alkylgruppe substituiert sein können, oder für gegebenenfalls durch Fluor, Chlor, Brom, Alkyl-, Alkenyl- oder Alkoxyreste mit bis zu 4 Kohlenstoffatomen, Trifluormethyl, Nitro, Cyan oder Alkoxycarbonyl mit bis zu 5 Kohlenstoffatomen substituierte Aralkylreste stehen oder $R^5$ mit $R^6$ Teil eines gegebenenfalls durch Methyl oder Ethyl substituierten Pyrrolidinium-, Piperidinium-, Hexamethyleniminium-, Morpholinium- oder Thiomorpholiniumrestes, der am quartären Stickstoff durch $R^4$ substituiert ist, und Y das Anion einer beliebigen, nicht phytotoxischen Säure HY bedeuten, fungizid stärker wirksam sind als Tetramethylthiuram-disulfid.

Als Substituenten für $R^1$, $R^2$ und $R^3$ in Formel I, kommen Halogen, wie Fluor, Chlor, Brom, Jod, Nitro, Cyano oder gegebenenfalls durch Halogen substituierte Alkyl- oder Alkoxyreste mit 1 bis 4 Kohlenstoffatomen, wie Methyl, Ethyl, Isopropyl, tert.-Butyl, Methoxy, Ethoxy, Trifluormethyl, Difluormethoxy, Tetrafluorethoxy, in Betracht.

A in Formel I bedeutet einen Chinuclidin- oder Pyrrolizidinbicyclus oder einen Rest der Formel $-N^{\oplus}R^4R^5R^6$. $R^4$, $R^5$ und $R^6$ stehen darin für Alkylgruppen mit bis zu 6 Kohlenstoffatomen, vorzugsweise mit bis zu 4 Kohlenstoffatomen, die durch Halogen, wie Fluor, Chlor, Brom, Alkoxy mit 1 bis 4 Kohlenstoffatomen, Cyano oder Dialkylamino mit 1 bis 4 Kohlenstoffatomen in einer Alkylgruppe substituiert sein können, beispielsweise Methyl, Ethyl, n-Propyl, Isopropyl, n-Butyl, Isobutyl, n-Pentyl, Isopentyl, n-Hexyl, Trifluormethyl, 2-Chlorethyl und 2-Bromethyl, für Allyl, 2-Butenyl oder 4-Chlor-2-butenyl, für Propargyl, für Cycloalkylreste mit bis zu 12 Kohlenstoffatomen oder Cycloalkenylreste mit bis zu 7 Kohlenstoffatomen, die durch Halogen, wie Fluor, Chlor, Brom, Alkoxy mit 1 bis 4 Kohlenstoffatomen, Cyano oder Dialkylamino mit 1 bis 4 Kohlenstoffatomen in einer Alkylgruppe substituiert sein können, beispielsweise Cyclohexyl, 2-Dimethylaminocyclohexyl und Cyclododecyl, oder für gegebenenfalls durch Fluor, Chlor, Brom, Alkyl-, Alkenyl- oder Alkoxyreste mit bis zu 4 Kohlenstoffatomen, Trifluormethyl, Nitro, Cyano oder Alkoxycarbonyl mit bis zu 5 Kohlenstoffatomen substituierte Aralkylreste, wie Benzyl, Phenethyl, Naphthylmethyl, beispielsweise 2-Fluorbenzyl, 4-Fluorbenzyl, 2-Chlorbenzyl, 4-Chlorbenzyl, 3-Brombenzyl, 4-Brombenzyl, 3-Trifluormethylbenzyl, 4-Trifluormethylbenzyl, 2-Methylbenzyl, 3-Methylbenzyl, 4-Methylbenzyl, 4-tert.-Butylbenzyl, 4-Nitrobenzyl, 2,4-Dichlorbenzyl, 3,4-Dichlorbenzyl, 2,4-Dimethylbenzyl, 3,4-Dimethylbenzyl, 4-Methoxybenzyl und 1-Naphthylmethyl.

Da die Wirkung der erfindungsgemässen Verbindungen der Formel I auf das Kation zurückzuführen ist, kann das Anion $Y^{\ominus}$ aus den nicht phytotoxischen Säuren beliebig gewählt werden. Y bedeutet beispielsweise Methylsulfonat, p-Dodecylbenzolsulfonat, Sulfat, Methosulfat, Nitrat, Phosphat, Jodid und insbesondere Chlorid und Bromid.

Bevorzugt sind Verbindungen der Formel I, in der $R^1$ Halogen, wie Fluor, Chlor oder Brom, eine unverzweigte oder verzweigte Alkyl- oder Alkoxygruppe mit 1 bis 4 Kohlenstoffatomen, wie Methyl, Ethyl, Isopropyl, tert.-Butyl, Methoxy, Ethoxy, Nitro oder Trifluormethyl, p und q, o, n 1, 2

oder 3, X Sauerstoff, m die Zahlen 2, 3 oder 4, A die Gruppe $-N^{\oplus}R^4R^5R^6$ und Y das Anion einer beliebigen, nicht phytotoxischen Säure HY bedeuten.

In der Gruppe $-N^{\oplus}R^4R^5R^6$ können $R^4$, $R^5$, $R^6$ gleich oder verschieden sein und unabhängig voneinander unverzweigte oder verzweigte, gegebenenfalls durch Halogen substituierte Alkylreste mit 1 bis 5 Kohlenstoffatomen, wie Methyl, Ethyl, n-Propyl, Isopropyl, n-Butyl, Isobutyl, n-Pentyl, Isopentyl, 2-Chlorethyl, 3-Chlorpropyl, 2-Bromethyl, 3-Brompropyl, 4-Brombutyl, Allyl, 2-Butenyl oder Cycloalkylreste mit 3 bis 6 Kohlenstoffatomen, wie Cyclopropyl, Cyclohexyl, bedeuten.

In einer weiteren Gruppe bevorzugter Verbindungen bedeutet $R^4$ gegebenenfalls durch Fluor, Chlor, Brom, Trifluormethyl, Nitro oder Alkyl mit 1 bis 4 Kohlenstoffatomen substituiertes Benzyl, wie Benzyl, 2-Fluorbenzyl, 4-Fluorbenzyl, 2-Chlorbenzyl, 4-Chlorbenzyl, 3-Brombenzyl, 4-Brombenzyl, 3-Trifluormethylbenzyl, 4-Trifluormethylbenzyl, 2-Methylbenzyl, 3-Methylbenzyl, 4-Methylbenzyl, 4-tert.-Butylbenzyl, 4-Nitrobenzyl, 2,4-Dichlorbenzyl, 3,4-Nitrobenzyl, 2,4-Dimethylbenzyl oder 3,4-Dimethylbenzyl, und $R^5$ ist mit $R^6$ Teil eines gegebenenfalls durch Methyl oder Ethyl substituierten 5-, 6- oder 7gliedrigen heterocyclischen Ringes mit 1 bis 3 Heteroatomen, beispielsweise eines Pyrrolidinium-, Piperidinium-, Hexamethyleniminium-, Morpholinium-, Thiomorpholinium-, 2,6-Dimethylmorpholinium, 2,6-Dimethyl-thiomorpholinium, 2-Ethyl-piperidinium-, 4-Methyl-piperidiniumringes.

Beispiele für Dibenzofuranderivate der Formel I sind:

N-Methyl-N-2-(7-bromdibenzofuran-3-oxy)-ethylpyrrolidiniumbromid,
N-Methyl-N-2-(7-bromdibenzofuran-3-oxy)-ethylpiperidiniumbromid,
N-Allyl-N-2-(7-bromdibenzofuran-3-oxy)-ethyl-)2′,6′-dimethylmorpholinium)-bromid,
N-Benzyl-N-2-(7-bromdibenzofuran-3-oxy)-ethylpyrrolidiniumbromid,
N-Benzyl-N-2-(7-bromdibenzofuran-3-oxy)-ethylpiperidiniumchlorid,
N-Benzyl-N-4-(7-bromdibenzofuran-3-oxy)-butyl-piperidiniumchlorid,
N-Methyl-N-4-(7-fluordibenzofuran-3-oxy)-butylpiperidiniumbromid,
N-Benzyl-N-2-(7-fluordibenzofuran-3-oxy)-ethylpiperidiniumchlorid,
N-Methyl-N-2-(7-chlordibenzofuran-3-oxy)-ethylpyrrolidiniumbromid,
N-Allyl-N-2-(7-chlor-dibenzofuran-3-oxy)-ethylpyrrolidiniumbromid,
N-Butyl-N-2-(7-chlordibenzofuran-3-oxy)-ethylpyrrolidiniumbromid,
N-Benzyl-N-2-(7-chlordibenzofuran-3-oxy)-ethylpyrrolidiniumchlorid,
N-Allyl-N-3-(7-chlor-dibenzofuran-3-oxy)-propylpiperidiniumbromid,
N-Allyl-N-3-(7-chlordibenzofuran-3-thio)-propylpiperidiniumbromid,
N-Methyl-N-4-(7-chlordibenzofuran-3-oxy)-butylpiperidiniumbromid,
N-Benzyl-N-3-(7-chlordibenzofuran-3-oxy)-propylpiperidiniumbromid,
N-Benzyl-N-4-(7-chlordibenzofuran-3-oxy)-butylammoniumbromid,
N-4-(7-Chlordibenzofuran-3-oxy)-butylpyrrolizidiniumbromid,
N-(1′-Buten-2′-yl-N-2-(7-chlordibenzofuran-3-oxy)-ethylpiperidiniumbromid,
N-Methyl-N-2-(7-chlordibenzofuran-3-oxy)-ethylhexamethylenimmoniumbromid,
N-Propyl-N-2-(7-chlordibenzofuran-3-oxy)-ethylhexamethylenimmoniumbromid,
N-Benzyl-N-2-(7-chlordibenzofuran-3-oxy)-ethylenhexamethylenimmoniumbromid,
N,N,N-Triethyl-N-2-(7-chlordibenzofuran-3-oxy)-ethylammoniumbromid,
N,N-Dimethyl-N-cyclohexyl-N-2-(7-chlordibenzofuran-3-oxy)-ethylammoniumbromid,
N,N-Dimethyl-N-(2′-dimethylamino)-cyclohexyl-N-2-(7-chlordibenzofuran-3-oxy)-ethylammoniumbromid,
N,N-Dimethyl-N-benzyl-N-2-(7-chlordibenzofuran-3-oxy)-ethylammoniumbromid,
N-(4′-Chlor-2′-buten-1′-yl)-N-2-(7-chlordibenzofuran-3-oxy)-ethylpiperidiniumchlorid,
N-Benzyl-N-2-(7-chlordibenzofuran-3-oxy)-ethyl-(2′,6′-dimethylmorpholin)-bromid,
N-benzyl-N-2-(7-chlordibenzofuran-3-oxy)-ethyl-(2′,6′-dimethylthiomorpholinium)-bromid,
N-Benzyl-N-2-(7-chlordibenzofuran-3-oxy)-ethylpiperidiniumchlorid,
N-(4′-Fluorbenzyl)-N-2-(7-chlordibenzofuran-3-oxy)-ethylpiperidiniumbromid,
N-(4′-Chlorbenzyl)-N-2-(7-chlordibenzofuran-3-oxy)-ethylpiperidiniumchlorid,
N-(4′-Methylbenzyl)-N-2-(7-chlordibenzofuran-3-oxy)-ethylpiperidiniumbromid,
N-(4′-Nitrobenzyl)-N-2-(7-chlordibenzofuran-3-oxy)-ethylpiperidiniumbromid,
N-(4′-Trifluormethylbenzyl)-N-2-(7-chlordibenzofuran-3-oxy)-ethylpiperidiniumbromid,
N-(4′-tert.-Butylbenzyl)-N-2-(7-chlordibenzofuran-3-oxy)-ethylpiperidiniumbromid,
N-(1-Naphthylmethyl)-N-2-(7-chlordibenzofuran-3-oxy)-ethylpiperidiniumchlorid,
N-Benzyl-N-2-(7-chlordibenzofuran-3-oxy)-ethyl(4′-methylpiperidinium)-chlorid,
N-3,4-Dimethylbenzyl)-N-2-(7-chlordibenzofuran-3-oxy)-ethyl-(4′-methylpiperidinium)-chlorid,
N-Benzyl-N-2-(7-chlordibenzofuran-3-oxy)-ethyl-(2′-ethylpiperidinium)-bromid,
N,N-Dimethyl-N-cyclododecyl-N-2-(6-trifluormethyl-dibenzofuran-3-oxy)-ethylammoniumbromid und
N-Methyl-N-4-(6,8-dichlor-7-methyldibenzofuran-3-oxy)-butylpiperidiniumbromid.

Man erhält die Dibenzofuranderivate der Formel I durch Umsetzung von

a) Verbindungen der Formel

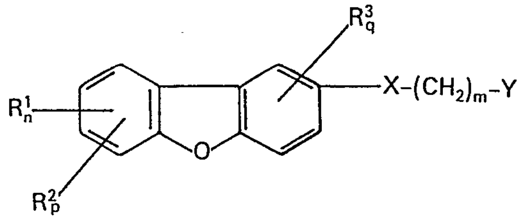

(II),

in der $R^1$, $R^2$, $R^3$, X, Y, m, n, p und q die oben angegebenen Bedeutungen haben, mit einem tertiären Amin der Formel A entsprechend den oben angegebenen Bedeutungen oder

b) Verbindungen der Formel

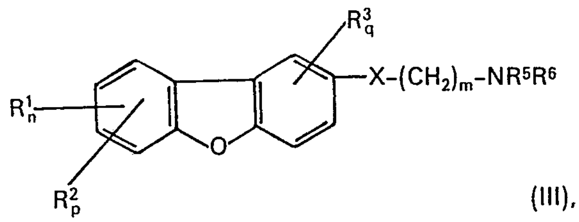

(III),

in der $R^1$, $R^2$, $R^3$, $R^5$, $R^6$, X, m, n, p und q die oben angegebenen Bedeutungen haben, mit einer Verbindung der Formel

$R^4$–Y (IV),

in der $R^4$ und Y die oben angegebenen Bedeutungen haben.

Die Reaktionen a) und b) werden gegebenenfalls in Gegenwart eines Lösungs- oder Verdünnungsmittels bei einer Temperatur im Bereich zwischen 20 und 150°C, vorzugsweise zwischen 30 und 140°C, durchgeführt. Der Ausgangsstoff der Formel II wird zweckmässigerweise mit dem 2- bis 10fachen molaren Überschuss des Amins der Formel A umgesetzt.

Als bevorzugte, gegenüber den Reaktionsteilnehmern inerte Lösungs- oder Verdünnungsmittel können beispielsweise aliphatische oder aromatische, gegebenenfalls halogenierte Kohlenwasserstoffe, wie Pentan, Cyclohexan, Benzol, Toluol, Xylol, Chlorbenzol, Dichlorbenzole, aliphatische Ketone, wie Aceton, Methylethylketon, Diethylketon oder Cyclopentanon, Ether, wie Diethylether, Dimethyloxyethan, Tetrahydrofuran oder Dioxan, Ester, wie Essigsäureethylester, Nitrile, wie Acetonitril, Amide, wie Dimethylformamid, Dimethylacetamid und N-Methylpyrrolidon, oder Gemische dieser Lösungsmittel verwendet werden.

Die Ausgangsstoffe der Formel II können leicht nach bekannten Verfahren hergestellt werden, beispielsweise durch Veretherung von Dibenzofuran-3-olen bzw. von Dibenzofuran-3-thiolen mit aliphatischen Dihalogeniden, wie 1,2-Dibromethan, 1,3-Dichlorpropan, 1-Chlor-3-brompropan, 1,3-Dibrompropan, 1-Chlor-4-brombutan oder 1,4-Dibrombutan, bevorzugt in siedendem Methylethylketon, Diethylketon oder Cyclopentanon in Gegenwart von mindestens äquivalenten Mengen Natriumcarbonat oder Kaliumcarbonat (Houben-Weyl, Methoden der Organischen Chemie, Band 6/3, S. 54–59, Georg Thieme-Verlag, Stuttgart, 1965). Alternativ können Dibenzofuran-3-oxyalkanole mit Thionylchlorid oder mit Phosphortribromid zu Verbindungen der Formel II umgesetzt werden (Rec. trav. Chim. 76, 129–146 (1957)).

Als tertiäre Amine der Formel A können z.B. Trimethylamin, Triethylamin, Tripropylamin, Methyl-diethylamin, Methyl-dipropylamin, Tributylamin, Tripentylamin, 1,2-Bis-(dimethylamino)-cyclohexan, N,N-Dimethyl-N-cyclododecylamin, N,N-Dimethyl-N-cyclohexylamin, N,N-Dimethyl-N-benzylamin, N-Methyl-pyrrolidin, N-Ethylpyrrolidin, N-Allyl-pyrrolidin, N-Propylpyrrolidin, N-Butylpyrrolidin, N-Methylpiperidin, N-2-Dimethylpiperidin, N-3-Dimethylpiperidin, N,N-Dimethylpiperidin, N-2,4-Trimethylpiperidin, N-3,5-Trimethylpiperidin, N-2,5-Trimethylpiperidin, N-Methyl-2-ethylpiperidin, N-Ethylpiperidin, N-Methyl-3-hydroxymethylpiperidin, N-Methylmorpholin, N-Ethylmorpholin, N-2,6-Trimethylmorpholin, N-Ethyl-2,6-dimethylmorpholin, N-Methyl-hexamethylenimin, N-Propyl-hexamethylenimin, Chinuclidin oder Pyrrolizidin eingesetzt werden.

Die tertiären Amine der Formel III lassen sich nach bekannten Verfahren herstellen, beispielsweise nach dem Schema

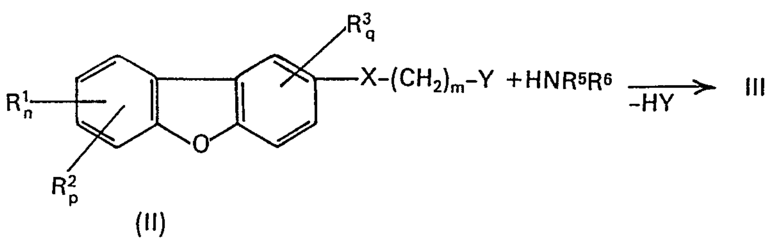


(II)

durch Alkylierung sekundärer Amine der Formel $NHR^5R^6$, in der $R^5$ und $R^6$ die obengenannten Bedeutungen haben, mit den Verbindungen der Formel II. Die Reaktionsbedingungen entsprechen hierbei denen der Umsetzung a).

Die bei der Umsetzung entstehende Säure HY kann leicht entfernt werden, z.B. durch Behandeln des Reaktionsgemisches mit wässrigen Lösungen von Alkalihydroxiden.

Als Amine der Formel $HNR^5R^6$ kommen bei-

spielsweise Dimethylamin, Dipropylamin, Diisopropylamin, Diallylamin, Dibutylamin, Diisobutylamin, N-Methylbenzylamin, Pyrrolidin, Piperidin, 2-Methylpiperidin, 3-Methylpiperidin, 4-Methylpiperidin, 2,4-Dimethylpiperidin, 3,5-Dimethylpiperidin, 2,6-Dimethylpiperidin, 2-Ethylpiperidin, N-Ethylbenzylamin, N-Butylbenzylamin, Morpholin, 2-Methylmorpholin, 3-Methylmorpholin, 2,6-Dimethylmorpholin, 2,5-Dimethylmorpholin, 2,6-Dimethylthiomorpholin oder Hexamethylenimin in Betracht.

Als Verbindungen der Formel IV können beispielsweise Methylchlorid, Methylbromid, Methyljodid, Dimethylsulfat, Ethylchlorid, Ethylbromid, Ethyljodid, Diethylsulfat, Dibromethan, 2-Methoxyethylbromid, Propylbromid, Propyljodid, 1-Chlor-3-brompropan, 1,3-Dibrompropan, Isopropylbromid, Allylchlorid, Allylbromid, n-Butylbromid, Butylchlorid, 1,4-Dibrombutan, 1,4-Dichlorbutan, Propargylchlorid, Propargylbromid, 1-Brom-2-buten, 1,4-Dichlor-2-buten, Benzylchlorid, Benzylbromid, 2-Fluorbenzylchlorid, 3-Fluorbenzylbromid, 4-Fluorbenzylbromid, 2-Chlorbenzylchlorid, 3-Chlorbenzylbromid, 4-Chlorbenzylbromid, 4-Brombenzylbromid, 2,4-Dichlorbenzylchlorid, 3,4-Dichlorbenzylchlorid, 4-Methylbenzylbromid, 2,4-Dimethylbenzylbromid, 3-Trifluormethylbenzylchlorid, 4-Trifluormethylbenzylbromid, 4-Nitrobenzylchlorid, 4-Nitrobenzylbromid, 4-tert.-Butylbenzylbromid, 3,4,5-Trimethoxybenzylchlorid, 4-Cyanbenzylchlorid und 1-Chlormethylchlorid und 1-Chlormethylnaphthalin eingesetzt werden.

Für die Herstellung von Ausgangsverbindungen der Formel II und III können beispielsweise die folgenden Dibenzofuran-3-ole und Dibenzofuran-3-thiole eingesetzt werden:

Dibenzofuran-3-ol, Dibenzofuran-3-thiol, 7-Fluor-dibenzofuran-3-ol, 7-Chlordibenzofuran-3-ol, 7-Chlordibenzofuran-3-thiol, 2,4,7-Trichlordibenzofuran-3-5-Chlordibenzofuran-3-ol, 6-Chlor-dibenzofuran-3-ol, 8-Chlordibenzofuran-3-ol, 5,7-Dichlor-benzofuran-3-ol, 6,7-Dichlor-dibenzofuran-3-ol, 7,8-Dichlordibenzofuran-3-ol, 7-Bromdibenzofuran-3-ol, 7-Brom-dibenzofuran-3-thiol, 7-Methyl-dibenzofuran-3-ol, 6,8-Dichlor-7-methyl-dibenzofuran-3-ol, 6-Trifluormethyl-dibenzofuran-3-ol, 7-Trifluor-methyl-dibenzofuran-3-ol, 7-Trifluormethyl-dibenzofuran-3-thiol, 7-tert.-Butyl-dibenzofuran-3-ol, 7-Methoxy-dibenzofuran-3-ol, 7-Chlor-2-nitro-dibenzofuran-3-ol und 7-Ethoxy-dibenzofuran-3-ol.

Die folgenden Beispiele erläutern die Herstellung der Verbindungen der Formel I.

Beispiel 1

Zu einer Lösung von 262 g (1,2 Mol) 7-Chlordibenzofuran-3-ol in 600 ml trockenem Dimethylformamid werden 83 g (0,6 Mol) Kaliumcarbonat zugegeben und anschliessend bei 100°C eine Lösung von 212 g (2,55 Mol) Ethylencarbonat in 300 ml Dimethylformamid in 90 Minuten zugetropft. Das Gemisch wird 8 Stunden bei 120°C gerührt, bei 100°C abgesaugt und das Filtrat wird eingeengt. Der Rückstand wird in 2,5 l Essigester gelöst, dreimal mit je 300 ml Wasser gewaschen, die organische Schicht getrocknet, mit 10 g Tierkohle entfärbt, auf 500 ml eingeengt und auf 10°C abgekühlt. Der Niederschlag wird abgesaugt und nacheinander bei +5°C mit 60 ml Methanol, 50 ml Ether und 200 ml Petrolether gewaschen und getrocknet. Man erhält 225 g 2-(7-Chlordibenzofuran-3-oxy)-ethanol vom Schmelzpunkt 118 bis 120°C; Ausbeute 71,4% d.Th.

Zu einer Suspension von 26,2 g (0,1 Mol) (2-(7-Chlordibenzofuranyl-3-oxy)-ethanol in 100 ml trockenem Toluol werden 11,9 g (0,15 Mol) Pyridin zugesetzt und anschliessend bei 3 bis 15°C 17,9 g (0,15 Mol) Thionylchlorid zugetropft. Das Gemisch wird 1 Tag bei 40°C und weitere 8 Stunden bei 70°C gerührt, abgekühlt und mit 150 ml Toluol und 200 ml Eiswasser versetzt. Die organische Schicht wird nacheinander mit 100 ml Wasser, 50 ml Salzsäure, 50 ml in Natronlauge und schliesslich mit 100 ml Wasser gewaschen, getrocknet und eingeengt. Der feste Rückstand wird mit 40 ml Petrolether bei +3°C (Eisbad) 30 Minuten verrührt, abgesaugt, mit wenig kaltem Petrolether gewaschen und getrocknet. Man erhält 22,8 g 1-Chlor-2-(7-chlordibenzofuranyl-3-oxy)-ethan als farblose Kristalle vom Schmelzpunkt 105 bis 107°C; Ausbeute 81% d.Th.

70,2 g (0,25 Mol) 1-Chlor-2-(7-chlordibenzofuran-3-oxy)-ethan und 150 g (1,76 Mol) Piperidin werden 8 Stunden bei 100°C und weitere 8 Stunden bei 140°C gerührt, abgekühlt und anschliessend unter vermindertem Druck eingeengt. Der Rückstand wird zwischen 100 ml Wasser und 400 ml Ether verteilt und unter Eiskühlung mit 100 ml 50%iger Natronlauge versetzt. Die Etherschicht wird abgetrennt, mit 100 ml Wasser gewaschen, getrocknet und unter vermindertem Druck eingeengt. Der feste Rückstand wird mit 50 ml n-Pentan bei +5°C 30 Minuten gerührt, abgesaugt und mit 30 ml kaltem Pentan gewaschen. Man erhält 70 g N-2-(7-Chloridbenzofuran-3-oxy)-ethyl-piperidin als farblose Kristalle; Schmelzpunkt 65 bis 67°C; Ausbeute 84% d.Th.

Eine Lösung von 11,5 g (0,035 Mol) N-2-(7-Chlordibenzofuran-3-oxy)-ethylpiperidin und 6,1 g (0,05 Mol) Allylbromid in 30 ml trockenem Dioxan und 30 ml trockenem Acetonitril wird 8 Stunden bei 80°C gerührt und auf 0°C abgekühlt. Der kristalline Niederschlag wird abgesaugt, mit 50 ml trockenem Ether gewaschen und unter vermindertem Druck getrocknet. Man erhält 13,5 g, entsprechend einer Ausbeute von 85,6% d.Th., N-Allyl-N-2-(7-chlordibenzofuran-3-oxy)-ethyl-piperidiniumbromid als weisse Kristalle vom Schmelzpunkt 189 bis 191°C. (Verbindung Nr. 1)

Beispiel 2

Eine Mischung aus 98,4 g (0,45 Mol) 7-Chlordibenzofuran-3-ol, 200 ml Methylethylketon, 93 g (0,67 Mol) Kaliumcarbonat und 283 g (1,4 Mol) Dibrompropan werden 24 Stunden unter Rückfluss gerührt. Nach Absaugen des anorganischen Niederschlages wird das Filtrat unter vermindertem Druck eingeengt. Der Rückstand wird in 500 ml Methylenchlorid aufgelöst, nacheinander mit 100 ml Wasser, 100 ml 2n Natronlauge und 100 ml Wasser gewaschen, über Natriumsulfat getrocknet und unter vermindertem Druck eingeengt. Der feste Rückstand wird mit 30 ml Pentan bei 0°C 30 Minuten stehen gelassen, abgesaugt und mit 30 ml kaltem n-Pentan gewaschen. Man erhält 77 g reines 1-Brom-3-(7-chlordibenzofuran-3-oxy)-propan vom Schmelzpunkt 83 bis 85°C; Ausbeute 50% d.Th.

Die Lösung von 14 g (0,041 Mol) 1-Brom-3-(7-chlordibenzofuran-3-oxy)-propan und 4,8 g (0,048 Mol) N-Methylpiperidin in 40 ml Dioxan und 40 ml Acetonitril werden 8 Stunden bei 70°C gerührt. Nach dem Abkühlen auf +10°C wird der gebildete, kristalline Niederschlag abgesaugt, mit 30 ml Ether und schliesslich mit 50 ml n-Pentan gewaschen und getrocknet. Man erhält 13,4 g, entsprechend einer Ausbeute von 74% d.Th., N-Methyl-N-3-(7-chlordibenzofuran-3-oxy)-propylpiperidiniumbromid vom Schmelzpunkt 212 bis 215°C (Verbindung Nr. 2)

Analog Beispiel 1 und 2 lassen sich beispielsweise folgende Verbindungen der Formel I herstellen:

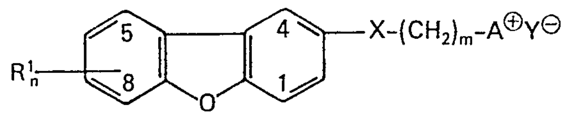

| Nr. | $R^1_n$ | m | X | $A^{\oplus}$ | $Y^{\ominus}$ | Fp[°C] |
|---|---|---|---|---|---|---|
| 3 | H | 2 | O | 1-Methyl-pyrrolidinium | $Br^{\ominus}$ | 158–160 |
| 4 | H | 2 | O | 1-Ethyl-pyrrolidinium | $Br^{\ominus}$ | 149–150 |
| 5 | H | 2 | O | 1-Allyl-pyrrolidinium | $Br^{\ominus}$ | 152–154 |
| 6 | H | 2 | O | 1-n-Propyl-pyrrolidinium | $Br^{\ominus}$ | 197–199 |
| 7 | H | 2 | O | 1-Methyl-piperidinium | $Br^{\ominus}$ | 191–193 |
| 8 | H | 2 | O | 1-Allyl-piperidinium | $Br^{\ominus}$ | 168–170 |
| 9 | 7–F | 2 | O | 1-Methyl-piperidinium | $Br^{\ominus}$ | 144–146 |
| 10 | 6–Cl | 2 | O | 1-Methyl-pyrrolidinium | $Br^{\ominus}$ | 191–192 |
| 11 | 6–Cl | 2 | O | 1-Methyl-piperidinium | $Br^{\ominus}$ | 173–175 |
| 12 | 6–Cl | 2 | O | N,N-Dimethyl-N-2-(2-dimethylamino)-cyclohexylammonium | $Br^{\ominus}$ | 244–246 |
| 13 | 6–Br | 2 | O | 1-Methyl-pyrrolidinium | $Br^{\ominus}$ | 181–183 |
| 14 | 7–Br | 2 | O | 1-Methyl-piperidinium | $Br^{\ominus}$ | 217–219 |
| 15 | 7–Br | 2 | O | 1-Allyl-2,6-dimethylmorpholinium | $Br^{\ominus}$ | 201–203 |
| 16 | 6–$CF_3$ | 2 | O | 1-Methyl-pyrrolidinium | $Br^{\ominus}$ | 218–220 |
| 17 | 6–$CF_3$ | 2 | O | 1-Methyl-piperidinium | $Br^{\ominus}$ | 192–194 |
| 18 | 6–$CF_3$ | 2 | O | N,N-Dimethylamino-cyclohexyl-ammonium | $Br^{\ominus}$ | 198–200 |
| 19 | 6–$CF_3$ | 2 | O | N,N-Dimethyl-N-cyclododecylammonium | $Br^{\ominus}$ | 209–213 |
| 20 | 7–Cl | 2 | O | 1-Methyl-pyrrolidinium | $Br^{\ominus}$ | Harz |
| 21 | 7–Cl | 2 | O | 1-Allyl-pyrrolidinium | $Br^{\ominus}$ | 152–154 |
| 22 | 7–Cl | 2 | O | 1-n-Butyl-pyrrolidinium | $Br^{\ominus}$ | 212–214 |
| 23 | 7–Cl | 3 | O | 1-Methyl-pyrrolidinium | $Br^{\ominus}$ | 154–157 |
| 24 | 7–Cl | 3 | O | 1-n-Butyl-pyrrolidinium | $Br^{\ominus}$ | 192–195 |
| 25 | 7–Cl | 4 | O | 1-Methyl-pyrrolidinium | $Br^{\ominus}$ | 165–167 |
| 26 | 7–Cl | 4 | O | 1-n-Butyl-pyrrolidinium | $Br^{\ominus}$ | 189–191 |
| 27 | 7–Cl | 3 | O | 1-Allyl-piperidinium | $Br^{\ominus}$ | 191–192 |
| 28 | 7–Cl | 3 | S | 1-Allyl-piperidinium | $Br^{\ominus}$ | 172–174 |
| 29 | 7–Cl | 4 | O | 1-Methyl-piperidinium | $Br^{\ominus}$ | 168–169 |
| 30 | 7–Cl | 4 | O | 1-Allyl-piperidinium | $Br^{\ominus}$ | 154–156 |
| 31 | 7–Cl | 4 | O | Pyrrolizidinium | $Br^{\ominus}$ | 144–147 |
| 32 | 7–Cl | 3 | O | N,N-Dimethyl-N-(2-dimethylamino)-cyclohexylammonium | $Br^{\ominus}$ | 182–184 |
| 33 | 7–Cl | 2 | O | 1-(2′-Buten-1′-yl)-piperidinium | $Br^{\ominus}$ | 197–199 |
| 34 | 7–Cl | 2 | O | 1-Benzyl-2,6-dimethyl-morpholinium | $Br^{\ominus}$ | 169–171 |
| 35 | 7–Cl | 2 | O | 1-Benzyl-2,6-dimethyl-thiomorpholinium | $Br^{\ominus}$ | 150–152 |
| 36 | 7–Cl | 2 | O | 1-Methyl-hexamethylen-iminium | $Br^{\ominus}$ | 198–200 |
| 37 | 7–Cl | 2 | O | 1-n-Propyl-hexamethylen-iminium | $Br^{\ominus}$ | 217–219 |
| 38 | 7–Cl | 2 | O | N,N,N-Triethylammonium | $Br^{\ominus}$ | 197–199 |
| 39 | 7–Cl | 2 | O | N,N-Dimethyl-N-cyclo-hexylammonium | $Br^{\ominus}$ | 143–145 |
| 40 | 7–Cl | 2 | O | N,N-Dimethyl-N-(2-dimethylamino)-cyclo-hexylammonium | $Br^{\ominus}$ | Harz |
| 41 | 7–Cl | 2 | O | N,N-Dimethyl-N-benzylammonium | $Br^{\ominus}$ | 193–195 |
| 42 | 7–Cl | 2 | O | 1-Benzyl-piperidinium | $Cl^{\ominus}$ | 201–203 |
| 43 | 7–Cl | 2 | O | 1-(4′-Fluorbenzyl)-piperidinium | $Br^{\ominus}$ | 209–211 |

| Nr. | $R^1_n$ | m | X | $A^{\oplus}$ | $Y^{\ominus}$ | Fp[°C] |
|---|---|---|---|---|---|---|
| 44 | 7-Cl | 2 | O | 1-(3′-Methylbenzyl)-piperidinium | $Br^{\ominus}$ | 202–204 |
| 45 | 7-Cl | 2 | O | 1-(2′,4′-Dichlorbenzyl)-piperidinium | $Cl^{\ominus}$ | 181–183 |
| 46 | 7-Cl | 2 | O | 1-(4′-Chlorbenzyl)-piperidinium | $Cl^{\ominus}$ | 185–186 |
| 47 | 7-Cl | 2 | O | 1-(4′-Nitrobenzyl)-piperidinium | $Br^{\ominus}$ | 136–138 |
| 48 | 7-Cl | 2 | O | 1-(4′-Trifluormethylbenzyl)-piperidinium | $Br^{\ominus}$ | 208–210 |
| 49 | 7-Cl | 2 | O | 1-(4′-tert.-Butylbenzyl)-piperidinium | $Br^{\ominus}$ | 204–206 |
| 50 | 7-Cl | 2 | O | 1-(1′-Naphthylmethyl)-piperidinium | $Cl^{\ominus}$ | 176–177 |
| 51 | 7-Cl | 2 | O | 1-(4′-Chlor-2′-buten-1′-yl)-piperidinium | $Cl^{\ominus}$ | Harz |
| 52 | 7-Cl | 2 | O | 1-Benzyl-4-methyl-piperidinium | $Cl^{\ominus}$ | 193–195 |
| 53 | 7-Cl | 2 | O | 1-Benzyl-2-ethylpiperidinium | $Br^{\ominus}$ | 157–159 |
| 54 | 7-Cl | 2 | O | 1-(1′-Naphthylmethyl)-4-methylpiperidinium | $Cl^{\ominus}$ | 165–168 |

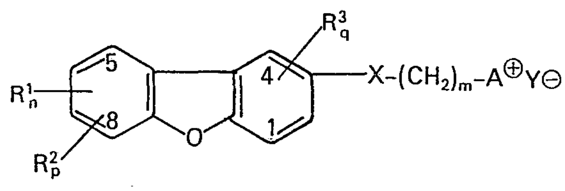

| Nr. | $R^1_n$ | m | X | $A^{\oplus}$ | $Y^{\ominus}$ | Fp[°C] |
|---|---|---|---|---|---|---|
| 55 | 6-Cl 7-Cl | 2 | O | 1-Methyl-pyrrolidinium | $Br^{\ominus}$ | 199–201 |
| 56 | 6-Cl 7-Cl | 2 | O | 1-Methyl-pyrrolidinium | $Br^{\ominus}$ | 211–213 |
| 57 | 6-Cl 7-Cl | 2 | O | 1-Methyl-pyrrolidinium | $Br^{\ominus}$ | 166–168 |
| 58 | 5-Cl 7-Cl | 2 | O | 1-Methyl-pyrrolidinium | $Br^{\ominus}$ | 119–121 |
| 59 | 5-Cl 7-Cl | 2 | O | 1-Methyl-pyrrolidinium | $Br^{\ominus}$ | 227–229 |
| 60 | 6-Cl, 7-$CH_3$, 8-Cl | 4 | O | 1-Methyl-piperidinium | $Br^{\ominus}$ | 245–247 |
| 61 | 7-Cl | 2 | O | 1-(3′,4′-Dichlorbenzyl)-piperidinium | $Cl^{\ominus}$ | 202–204 |
| 62 | 7-Cl | 2 | O | 1-(2′,4′-Dichlorbenzyl)-hexamethyleniminium | $Cl^{\ominus}$ | 186–188 |
| 63 | 7-Cl | 2 | O | 1-(2′,4′-Dichlorbenzyl)-2-ethylpiperidinium | $Cl^{\ominus}$ | 149–152 |
| 64 | 7-Cl | 2 | O | 1-(2′,4′-Dichlorbenzyl)-4-methylpiperidinium | $Cl^{\ominus}$ | 185–187 |
| 65 | 7-Cl | 2 | O | 1-(4′-Brombenzyl)-piperidinium | $Cl^{\ominus}$ | 210–212 |
| 66 | 7-Cl | 2 | O | 1-(2′Chlor-4′-nitrobenzyl)-piperidinium | $Cl^{\ominus}$ | 202–204 |
| 67 | 7-Cl | 2 | O | N-Methyl-N-benzyl-N-(4′-tert.-butylbenzyl)-ammonium | $Cl^{\ominus}$ | 153–155 |
| 68 | 7-Cl | 2 | O | 1-(4′-tert.-Butylbenzyl)-hexamethyleniminium | $Cl^{\ominus}$ | 135–137 |
| 69 | 7-Cl | 2 | O | 1-(4′-tert.-Butylbenzyl)-4-methylpiperidinium | $Cl^{\ominus}$ | 195–197 |
| 70 | 7-Cl | 2 | O | 1-(4′-tert.-Butylbenzyl)-2,6-dimethylmorpholinium | $Cl^{\ominus}$ | 135–138 |
| 71 | 7-Cl | 3 | O | 1-(4′-tert.-Butylbenzyl)-piperidinium | $Cl^{\ominus}$ | 225–227 |

Die neuen Wirkstoffe zeigen eine starke fungitoxische Wirksamkeit gegen phytopathogene Pilze. Sie dienen insbesondere zur Verhütung und Heilung von Pflanzenkrankheiten, die durch Mikroorganismen verursacht werden, wie z.B. Botrytis begonie, Botrytis cinerea, Plasmopara viticola, Monilia fructigena, Alternaria solani, Sclerotinia sclerotiorum, Piricularia oryzae, Pellicularia filamentosa, Erysiphe graminis, Erysiphe cichoriacearum, Chaetomium globosum, Sclerotinia cinerea, Aspergillus niger, Xanthomonas oryzae, Xanthomonas citri.

Die erfindungsgemässen Wirkstoffe können gleichzeitig das Wachstum von zwei oder mehr der genannten Pilze unterdrücken und besitzen eine hohe Planzenverträglichkeit. Ein Teil der Wirkstoffe zeigt kurative Eigenschaften, d.h. die Anwendung der Mittel kann noch nach erfolgter Infektion der Planzen durch die Krankheitserreger vorgenommen werden, um einen sicheren Bekämpfungserfolg zu erzielen.

Die fungiziden Mittel enthalten 0,1 bis 95% (Gewichtsprozent) Wirkstoff, vorzugsweise 0,5 bis 90%. Die Aufwandmengen liegen je nach Art des gewünschten Effektes zwischen 0,1 und 5 kg Wirkstoff je ha.

Die erfindungsgemässen Wirkstoffe können auch zusammen mit anderen Wirkstoffen, z.B. Herbiziden, Insektiziden, Wachstumsregulatoren und anderen Fungiziden, oder auch mit Düngemitteln vermischt und ausgebracht werden. In vielen Fällen erhält man bei der Mischung mit Fungiziden auch eine Vergrösserung des fungiziden Wirkungsspektrums; bei einer Anzahl dieser Fungizidmischungen treten auch synergistische Effekte auf, d.h. die fungizide Wirksamkeit des Kombinationsproduktes ist grösser als die der addierten Wirksamkeiten der Einzelkomponenten. Eine besonders günstige Vergrösserung des Wirkungsspektrums wird mit folgenden Fungiziden erzielt:

Manganethylenbisdithiocarbamat
Mangan-Zinkethylenbisdithiocarbamat
Ammoniak-Komplex von Zink-(N,N-ethylen-bis-dithiocarbamat)
N-Trichlormethylthio-tetrahydrophthalimid
N-Trichlormethyl-phthalimid
5-Äthoxy-3-trichlormethyl-1,2,3-thiadiazol
2-Methoxycarbonylamino-benzimidazol
2-Rhodanmethylthiobenzthiazol
1,4-Dichlor-2,5-dimethoxybenzol
2,3-Dichlor-6-methyl-1,4-oxathiin-5-carbonsäureanilid
2-Methyl-5,6-dihydro-4-H-pyran-3-carbonsäure-anilid
2,4,5-Trimethyl-furan-3-carbonsäureanilid
2-Methyl-furan-3-carbonsäureanilid
2,5-Dimethyl-furan-3-carbonsäurecyclohexyl-amid
N-Cyclohexyl-N-methoxy-2,5-dimethyl-furan-3-carbonsäureamid
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion.

Die erfindungsgemässen Verbindungen können aber auch mit folgenden Fungiziden kombiniert werden:

Dithiocarbamate und deren Derivate, wie
Ferridimethyldithiocarbamat
Zinkdimethyldithiocarbamat
Zinkethylenbisdithiocarbamat
Tetramethylthiuramdisulfide
Zink-(N,N-propylen-bis-dithiocarbamat)
Ammoniak-Komplex von Zink-(N,N'-propylen-bis-dithiocarbamat) und
N,N'-Polypropylen-bis-(thiocarbamoyl)-disulfid
Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
heterocyclische Strukturen, wie
2-Heptadecyl-2-imidazolin-acetat
2,4-Dichlor-6-(o-chloranilino)-s-triazin
0,0-Diethyl-phthalimidophonothioat
5-Amino-1-(bis-(dimethylamino)-phosphinyl)-3-phenyl-1,2,4-triazol)
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin-4,4-dioxid
2,3-Dihydro-5-carboxanilido-6-methyl-1,4-oxathiin
2-(Furyl-(2))-benzimidazol
Piperazin-1,4-diylbis-(1-(2,2,2-trichlor-ethyl)-formamid
2-(Thiazolyl-(4)-benzimidazol
5-Butyl-2-dimethylamino-4-hydroxy-6-methyl-pyrimidin
Bis-(p-chlorphenyl)-3-pyridinmethanol
1,2-Bis--(3-ethoxycarbonyl-2-thioureido)-benzol
1,2-Bis-(3-methoxycarbonyl-2-thioureido)-benzol
sowie verschiedene Fungizide, wie
Dodecylguanidinacetat
3-(3-(3,5-Dimethyl-2-oxycyclohexyl)-2-hydroxyethyl)-glutarimid
Hexachlorbenzol
N-Dichlorfluormethylthio-N',N'-dimethyl-N-phenyl-schwefelsäurediamid
2,5-Dimethyl-furan-3-carbonsäureanilid
2-Methyl-benzoesäure-anilid
2-Jod-benzoesäure-anilid
1-(3,4-Dichloranilino(-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
α-(2-Chlorphenyl)-α-(4-chlorphenyl)-5-pyrimidin-methanol.

Die neuen Wirkstoffe werden beispielsweise in Form von direkt versprühbaren Lösungen, Pulvern, Suspensionen, auch hochprozentige wässrige, ölige oder sonstige Suspensionen oder Dispersionen, Emulsionen, Öldispersionen, Pasten, Stäubemitteln, Streumitteln, Granulaten durch Versprühen, Vernebeln, Verstäuben, Verstreuen oder Giessen ausgebracht. Die Anwendungsformen richten sich ganz nach den Verwendungszwecken; sie sollten in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser usw. in Betracht.

Wässrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalz, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäure mit Phenol und Formaldehyd, Polyoxyethylen-octylphenolether, ethoxyliertes Isooctylphenol-, Octylphenol-, Nonylphenol, Alkylphenolpolyglykolether, Tributylphenylpolylglykolether, Alkalarylpolyätheralkohole, Isotridecylalkohol, Fettalkoholäthylenoxid-Kondensate, ethoxyliertes Rizinusöl, Polyoxyäthylenalkylether, ethoxyliertes Polyoxypropylen, Laurylalkoholpolylglykoletheracetal, Sorbitester, Lignin, Sulfitablaugen und Methylcellulose in Betracht. Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löss, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nussschalenmehl, Cellulosepulver und andere feste Trägerstoffe.

Beispiele für solche Zubereitungen sind:

I. Man vermischt 90 Gewichtsteile der Verbindung des Beispiels 1 mit 10 Gewichtsteilen N-Methyl-pyrrolidon und erhält eine Lösung, die zur Anwendung in Form kleinster Tropfen geeignet ist.

II. 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgiessen und feines Verteilen der Lösung in 100.000 Gewichtsteilen Wasser erhält man eine wässrige Dispersion, die 0,02 Gew.-% des Wirkstoffs enthält.

III. 20 Gewichtsteile der Verbindung Nr. 13 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingiessen und feines Verteilen der Lösung in 100.000 Gewichtsteilen Wasser erhält man eine wässrige Dispersion, die 0,02 Gew.-% des Wirkstoffs enthält.

IV. 20 Gewichtsteile der Verbindung Nr. 14 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingiessen und feines Verteilen der Lösung in 100.000 Gewichtsteilen Wasser erhält man eine wässrige Dispersion, die 0,02 Gew.-% des Wirkstoffs enthält.

V. 20 Gewichtsteile der Verbindung Nr. 19 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 200.000 Gewichtsteilen Wasser erhält man eine Spritzbrühe, die 0,1 Gew.-% des Wirkstoffs enthält.

VI. 5 Gewichtsteile der Verbindung Nr. 25 werden mit 95 Gewichtsteilen feinteiligem Kaolin innig vermischt. Man erhält auf diese Weise ein Stäubemittel, das 5 Gew.-% des Wirkstoffs enthält.

VII. 30 Gewichtsteile der Verbindung Nr. 37 werden mit einer Mischung aus 92 Gewichtsteilen pulverförmigem Kieselsäuregel und 8 Gewichtsteilen Paraffinöl, das auf die Oberfläche dieses Kiselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 42 werden mit 30 Teilen Natriumsalz eines Phenolsulfonsäure-harnstoff-formaldehyd-Kondensates, 2 Teilen Kieselgel und 48 Teilen Wasser innig vermischt. Man erhält eine stabile Dispersion.

IX. 20 Teile der Verbindung Nr. 51 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die folgenden Beispiele belegen die biologische Wirkung der neuen Verbindungen. Vergleichsmittel ist der bekannte, insbesondere zur Bekämpfung von Botrytis geeignete Wirkstoff Tetramethylthiuramdisulfid (Chem. Week 111 (4), 39 (1972)).

Beispiel A

Wirksamkeit gegen Botrytis cinerea an Paprika

Paprikasämlinge der Sorte «Neusiedler Ideal Elite» werden, nachdem sich 4 bis 5 Blätter gut entwickelt haben, mit 0,025 gewichtsprozentigen

wässrigen Suspensionen, die 80% Wirkstoff und 20% Natriumligninsulfonat in der Trockensubstanz enthalten, tropfnass gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt, um optimale Bedingungen für die Pilzentwicklung zu erhalten. Nach 5 Tagen hat sich die Krankheit auf den unbehandelten Kontrollpflanzen so stark entwickelt, dass die entstandenen Blattnekrosen den überwiegenden Teil der Blätter bedecken.

In diesem Test zeigen die Wirkstoffe Nr. 1, 2, 13, 14, 15, 16, 20, 22, 23, 25, 27, 29, 31, 32, 33, 36, 37, 38, 40, 41, 42, 45, 58, 60 eine bessere Wirkung als das Vergleichsmittel.

Beispiel B

Wirksamkeit gegen Gurkenmehltau

Blätter von in Töpfen gewachsenen Gurkenkeimlingen werden mit wässrigen Emulsionen aus 80% Wirkstoff und 20% Emulgiermittel besprüht und nach dem Antrocknen des Spritzbelages mit Oidien (Sporen) des Gurkenmehltaus (Erysiphe cichoreacearum) bestäubt. Die Versuchspflanzen werden anschliessend im Gewächshaus bei Temperaturen zwischen 20 und 22°C und 75 bis 80% relativer Luftfeuchtigkeit aufgestellt. Nach 10 Tagen wird das Ausmass der Mehltaupilzentwicklung ermittelt.

In diesem Test zeigen die Wirkstoffe Nr. 13, 14, 15, 19, 31, 32, 36, 41, 42, 45, 57, 59, 60 eine sehr gute Wirkung.

Beispiel C

Wirksamkeit gegen Bakterien

Zur Ermittlung der Wirksamkeit gegenüber Bakterien werden in sterilen Reagenzgläsern je 5 ml steigender Verdünnungen der Wirkstoffe zu 5 ml doppelt konzentrierter Nährbouillon gegeben und vermischt. Durch Zugabe von einem Tropfen einer 1:10 verdünnten, 16 Stunden alten Bouillon-Kultur der Bakterien-Arten Staphylococcus aureus bzw. Escherichia coli werden dann die Röhrchen beimpft und 24 Stunden bei 37°C bebrütet. Nach dieser Zeit werden Proben aus den Röhrchen auf Bakteriennährböden übertragen und diese ebenfalls 24 Stunden lang bei 37°C bebrütet. Diejenige Verdünnungsstufe, bei welcher nach dem Übertragen einer Probe auf den Nährboden keine Bakterienentwicklung mehr erfolgt, wird als Abtötungswert angegeben.

In diesem Test zeigen die Wirkstoffe Nr. 53, 54, 55, 58 und 60 eine gute Wirkung.

Beispiel D

Wirksamkeit gegen Candida albicans und Oidium lactis

Zur Prüfung der Wirksamkeit gegenüber Pilzen werden die Wirkstoffe einer für das Wachstum der Pilze Candida albicans bzw. Oidium lactis optimalen Nährlösung in Mengen von 100, 50, 25, 12, 6 und 3 Gewichtsteilen pro Million Teile Nährlösung zugesetzt. Jeweils 10 ml Nährlösungswirkstoffgemisch werden in sterile Reagenzgläser gegeben und mit einem Tropfen einer Sporensuspension beimpft, die $10^6$ Konidien bzw. Zellen enthält. nach 120-stündiger Bebrütung werden aus denjenigen Röhrchen, die kein sichtbares Pilzwachstum zeigen, Proben entnommen und auf Pilznährböden übertragen.

In diesem Test zeigen die Wirkstoffe Nr. 54, 55, 58 und 60 eine gute Wirkung.

## Patentansprüche

1. Dibenzofuranderivate der Formel

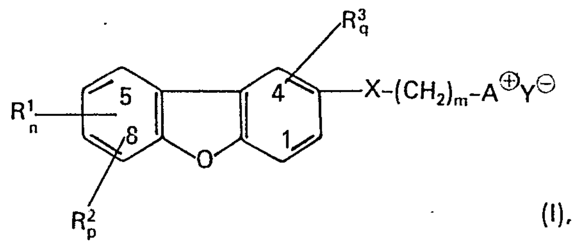

(I),

in der

$R^1$, $R^2$ und $R^3$ gleich oder verschieden sind und Halogen, eine gegebenenfalls halogensubstituierte Alkyl- oder Alkoxygruppe mit 1 bis 4 Kohlenstoffatomen, Cyan oder Nitro,

n, p und q die Zahlen 0, 1, 2 oder 3,

X Sauerstoff oder Schwefel,

m die Zahlen 2, 3, oder 4,

A einen Chinuclidin- oder Pyrrolizidinbicyclus oder die Gruppe $-N^{\oplus}R^4R^5R^6$,

in der $R^4$, $R^5$ und $R^6$ gleich oder verschieden sind und unabhängig voneinander für Alkylreste mit bis zu 6 Kohlenstoffatomen, Allyl, 2-Butenyl, 4-Chlor-2-butenyl, Propargyl, für Cycloalkylreste mit bis zu 12 Kohlenstoffatomen oder Cycloalkenylreste mit bis zu 7 Kohlenstoffatomen, wobei diese acyclischen und cyclischen Reste durch Halogen, Cyan, Alkoxy mit 1 bis 4 Kohlenstoffatomen oder Dialkylamino mit 1 bis 4 Kohlenstoffatomen in einer Alkylgruppe substituiert sein können, oder für gegebenenfalls durch Fluor, Chlor, Brom, Alkyl-, Alkenyl- oder Alkoxyreste mit bis zu 4 Kohlenstoffatomen, Trifluormethyl, Nitro, Cyan oder Alkoxycarbonyl mit bis zu Kohlenstoffatomen substituierte Aralkylreste stehen oder $R^5$ mit $R^6$ eines gegebenenfalls durch Methyl oder Ethyl substituierten Pyrrolidinium-, Piperidinium-, Hexamethyleniminium-, Morpholinium- oder Thiomorpholiniumrestes ist, und Y das Anion einer beliebigen, nicht phytotoxischen Säure HY bedeuten.

2. Dibenzofuranderivate der Formel I gemäss Anspruch 1, dadurch gekennzeichnet, das $R^1$ Halogen, eine Alkyl- oder Alkoxygruppe mit 1 bis 4 Kohlenstoffatomen, Trifluormethyl oder Nitro,

n die Zahlen 1, 2 oder 3,

p und q 0,

X Sauerstoff,

m die Zahlen 2, 3 oder 4,

A die Gruppe $-N^{\oplus}R^4R^5R^6$,

in der $R^4$, $R^5$ und $R^6$ gleich oder verschieden sind und unabhängig voneinander für gegebenenfalls

durch Halogen substituierten Alkylreste mit 1 bis 5 Kohlenstoffatomen oder für Cycloalkylreste mit 3 bis 6 Kohlenstoffatomen stehen, und
Y das Anion einer beliebigen, nicht phytotoxischen Säure NY bedeuten.

3. Dibenzofuranderivate der Formel I gemäss Anspruch 1, dadurch gekennzeichnet, dass $R^1$ Halogen, eine Alkyl- oder Alkoxygruppe mit 1 bis 4 Kohlenstoffatomen, Trifluormethyl oder Nitro,
n die Zahl 1,
p und q 0,
X Sauerstoff,
m die Zahlen 2, 3 oder 4,
A die Gruppen $-N^{\oplus}R^4R^5R^6$,
in der $R^4$ für gegebenenfalls durch Fluor, Chlor, Brom, Trifluormethyl, Nitro oder Alkyl mit 1 bis 4 Kohlenstoffatomen substituiertes Benzyl und $R^5$ mit $R^6$ Teil eines gegebenenfalls durch Methyl oder Ethyl substituierten Pyrrolidinium- Piperidinium-, Hexamethyleniminium-, Morpholinium- oder Thiomorpholiniumrestes ist und
Y das Anion einer beliebigen, nicht phytotoxischen Säure HY bedeuten.

4. Verfahren zur Herstellung von Dibenzofuranderivaten der Formel I gemäss Anspruch 1, dadurch gekennzeichnet, dass man

a) Verbindungen der Formel

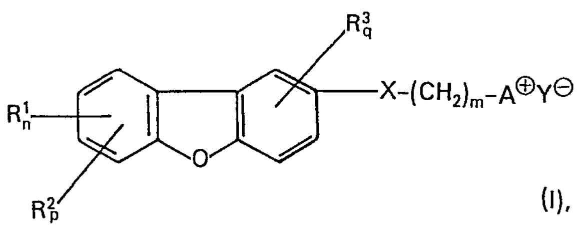

(I),

in der
$R^1$, $R^2$, $R^3$, X, Y, m, n, p und q die im Anspruch 1 angegebenen Bedeutungen haben, mit einem tertiären Amin der Formel A entsprechend den im Anspruch 1 angegebenen Bedeutungen oder

b) Verbindungen der Formel

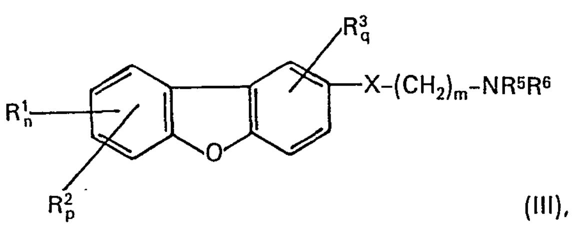

(III),

in der
$R^1$, $R^2$, $R^3$, $R^5$, $R^6$, X, m, n, p und q die im Anspruch 1 angegebenen Bedeutungen haben, mit einer Verbindung der Formel

$R^4$-Y (IV),

in der $R^4$ und Y die im Anspruch 1 angegebenen Bedeutungen haben, umsetzt.

5. Fungizides Mittel, enthaltend inerte Zusatzstoffe und ein Dibenzofuranderivat der Formel

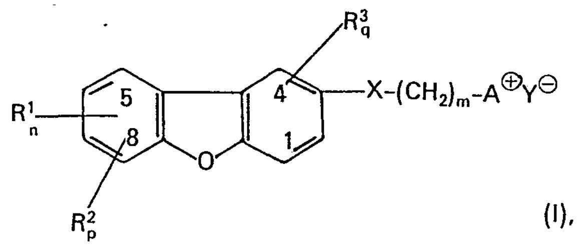

(I),

in der
$R^1$, $R^2$ und $R^3$ gleich oder verschieden sind und Halogen, eine gegebenenfalls halogensubstituierte Alkyl- oder Alkoxygruppe mit 1 bis 4 Kohlenstoffatomen, Cyan oder Nitro,
n, p und q die Zahlen 0, 1, 2 oder 3,
X Sauerstoff oder Schwefel,
m die Zahlen 2, 3 oder 4,
A einen Chinuclidin- oder Pyrrolizidinbicyclus oder die Gruppe $-N^{\oplus}R^4R^5R^6$,
in der $R^4$, $R^5$ und $R^6$ gleich oder verschieden sind und unabhängig voneinander für Alkylreste mit bis zu 6 Kohlenstoffatomen, Allyl, 2-Butenyl, 4-Chlor-2-butenyl, Propargyl, für Cycloalkylreste mit bis zu 12 Kohlenstoffatomen oder Cycloalkenylreste mit bis zu 7 Kohlenstoffatomen, wobei diese acylischen und cyclischen Reste durch Halogen, Cyan, Alkoxy mit 1 bis 4 Kohlenstoffatomen oder Dialkylamino mit 1 bis 4 Kohlenstoffatomen in einer Alkylgruppe substituiert sein können, oder für gegebenenfalls durch Fluor, Chlor, Brom, Alkyl-, Alkenyl- oder Alkoxyreste mit bis zu 4 Kohlenstoffatomen, Trifluormethyl, Nitro, Cyan oder Alkoxycarbonyl mit bis zu 5 Kohlenstoffatomen substituierte Aralkylreste stehen oder $R^5$ mit $R^6$ Teil eines gegebenenfalls durch Methyl oder Ethyl substituierten Pyrrolidinium-, Piperidinium-, Hexamethyleniminium-, Morpholinium- oder Thiomorpholiniumrestes ist, und
Y das Anion einer beliebigen, nicht phytotoxischen Säure HY bedeuten, als Wirkstoff.

6. Fungizides Mittel, enthaltend inerte Zusatzstoffe und ein Dibenzofuranderivat der Formel I gemäss Anspruch 1, in der $R^1$ Halogen, eine Alkyl- oder Alkoxygruppe mit 1 bis 4 Kohlenstoffatomen,
Trifluormethyl oder Nitro,
n die Zahlen 1, 2 oder 3,
p und q 0,
X Sauerstoff,
m die Zahlen 2, 3 oder 4,
A die Gruppe $-N^{\oplus}R^4R^5R^6$,
in der $R^4$, $R^5$ und $R^6$ gleich oder verschieden sind und unabhängig voneinander für gegebenenfalls durch Halogen substituierte Alkylreste mit 1 bis 5 Kohlenstoffatomen oder für Cycloalkylreste mit 3 bis 6 Kohlenstoffatomen stehen und
Y das Anion einer beliebigen, nicht phytotoxischen Säure HY bedeuten, als Wirkstoff.

7. Fungizides Mittel, enthaltend inerte Zusatzstoffe und ein Dibenzofuranderivat der Formel I gemäss Anspruch 1, in der $R^1$ Halogen, eine Alkyl- oder Alkoxygruppe mit 1 bis 4 Kohlenstoffatomen,

Trifluormethyl oder Nitro,
n die Zahl 1,
p und q 0,
X Sauerstoff,
m die Zahlen 2, 3 oder 4,
A die Gruppe $-N^{\oplus}R^4R^5R^6$,
in der $R^4$ für gegebenenfalls durch Fluor, Chlor, Brom, Trifluormethyl, Nitro oder Alkyl mit 1 bis 4 Kohlenstoffatomen substituiertes Benzyl und $R^5$ mit $R^6$ Teil eines gegebenenfalls durch Methyl oder Ethyl substituierten Pyrrolidinium-, Piperidinium-, Hexamethyleniminium-, Morpholinium- oder Thiomorpholiniumrestes ist, und
Y das Anion einer beliebigen, nicht phytotoxischen Säure HY bedeuten, als Wirkstoff.

8. Verfahren zur Bekämpfung von Pilzen, dadurch gekennzeichnet, dass man ein Dibenzofuranderivat der Formel I gemäss Anspruch 1 auf diese bzw. auf durch Pilzbefall bedrohte Flächen, Pflanzen oder Saatgüter einwirken lässt.

**Claims**

1. A dibenzofuran derivative of the formula

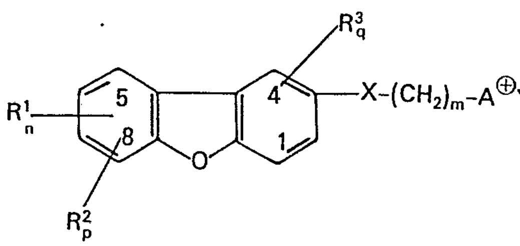

(I),

where $R^1$, $R^2$ and $R^3$ are identical or different and each is halogen, unsubstituted or halogen-substituted alkyl or alkoxy of 1 to 4 carbon atoms, cyano or nitro, n, p and q are 0, 1, 2 or 3, X is oxygen or sulfur, m is 2, 3 or 4, $A^{\oplus}$ is quinuclidinium or pyrrolizidinium, or $-N^{\oplus}R^4R^5R^6$, where $R^4$, $R^5$ and $R^6$ are identical or different and each independently of one another is alkyl of up to 6 carbon atoms, allyl, buten-2-yl, 4-chlorobuten-2-yl, propargyl, or cycloalkyl of up to12 carbon atoms or cycloalkenyl of up to 7 carbon atoms, which acyclic and cyclic radicals may be substituted by halogen, cyano, alkoxy of 1 to 4 carbon atoms or dialkylamino, where alkyl is of 1 to 4 carbon atoms, or is aralkyl which is unsubstituted or substituted by fluorine, chlorine, bromine, alkyl, alkenyl or alkoxy of up to 4 carbon atoms, trifluoromethyl, nitro, cyano or alkoxycarbonyl of up to 5 carbon atoms, or $R^5$ together with $R^6$ is part of a pyrrolidinium, piperidinium, hexamethyleneiminium, morpholinium or thiomorpholinium ring which may be substituted by methyl or ethyl, and $Y^{\ominus}$ is the anion of any desired non-phytotoxic acid HY.

2. A dibenzofuran derivative of the formula I as claimed in claim 1, wherein $R^1$ is halogen, alkyl or alkoxy of 1 to 4 carbon atoms, trifluoromethyl or nitro, n is 1, 2 or 3, p and q are 0, X is oxygen, m is 2, 3 or 4, A is $-N^{\oplus}R^4R^5R^6$, $R^4$, $R^5$ and $R^6$ being identical or different and each independently of one another being unsubstituted or halogen-substituted alkyl of 1 to 5 carbon atoms or cycloalkyl of 3 to 6 carbon atoms, and $Y^{\ominus}$ is the anion of any desired non-phytotoxic acid HY.

3. A dibenzofuran derivative of the formula I as claimed in claim 1, wherein $R^1$ is halogen, alkyl or alkoxy of 1 to 4 carbon atoms, trifluoromethyl or nitro, n is 1, p and q are 0, X is oxygen, m is 2, 3 or 4, A is $-N^{\oplus}R^4R^5R^6$, $R^4$ being unsubstituted benzyl or benzyl substituted by fluorine, chlorine, bromine, trifluoromethyl, nitro or by alkyl of 1 to 4 carbon atoms, and $R^5$ together with $R^6$ being part of a pyrrolidinium, piperidinium, hexamethyleneiminium, morpholinium or thiomorpholinium ring which may be substituted my methyl or ethyl, and $Y^{\ominus}$ is the anion of any desired non-phytotoxic acid HY.

4. A process for the preparation of a dibenzofuran derivative of the formula I as claimed in claim 1, wherein

(a) a compound of the formula

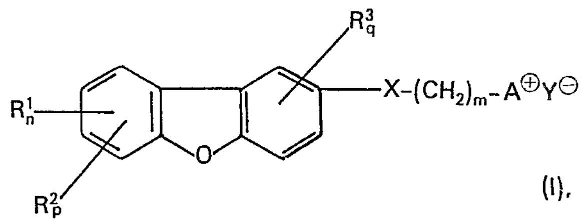

(I),

where $R^1$, $R^2$, $R^3$, X, Y, m, n, p and q have the meanings given in claim 1, is reacted with a tertiary amine of the formula A having the meanings given in claim 1, or

(b) a compound of the formula

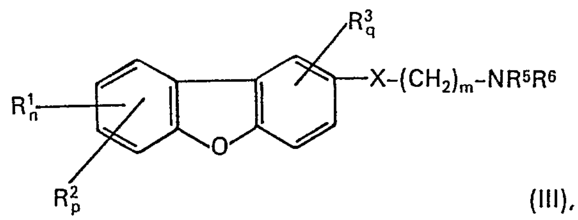

(III),

where $R^1$, $R^2$, $R^3$, $R^5$, $R^6$, X, m, n, p and q have the meanings given in claim 1, is reacted with a compound of the formula

$R^4$-Y (IV)

where $R^4$ and Y have the meanings given in claim 1.

5. A fungicidal agent containing inert additives and, as active ingredient, a dibenzofuran derivative of the formula

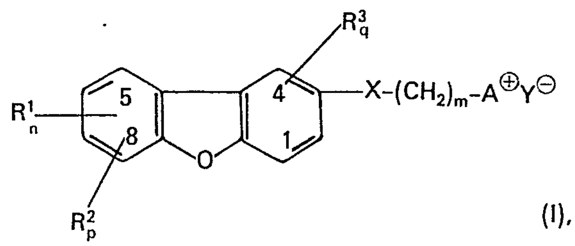

(I),

where $R^1$, $R^2$ and $R^3$ are identical or different and each is halogen, unsubstituted or halogen-substituted alkyl or alkoxy of 1 to 4 carbon atoms, cyano or nitro, n, p and q are 0, 1, 2 or 3, X is oxygen or sulfur, m is 2, 3 or 4, $A^{\oplus}$ is quinuclidinium or pyrrolizidinium, or $-N^{\oplus}R^4R^5R^6$, where $R^4$, $R^5$ and $R^6$ are identical or different and each independently of one another is alkyl of up to 6 carbon atoms, allyl, buten-2-yl, 4-chlorobuten-2-yl, propargyl, or cycloalkyl of up to 12 carbon atoms or cycloalkenyl of up to 7 carbon atoms, which acyclic and cyclic radicals may be substituted by halogen, cyano, alkoxy of 1 to 4 carbon atoms or dialkylamino, where alkyl is of 1 to 4 carbon atoms, or is aralkyl which is unsubstituted or substituted by fluorine, chlorine, bromine, alkyl, alkenyl or alkoxy of up to 4 carbon atoms, trifluoromethyl, nitro, cyano or alkoxycarbonyl of up to 5 carbon atoms, or $R^5$ together with $R^6$ is part of a pyrrolidinium, piperidinium, hexamethyleneiminium, morpholinium or thiomorpholinium ring which may be substituted by methyl or ethyl, and $Y^{\ominus}$ is the anion of any desired non-phytotoxic acid HY.

6. A fungicidal agent containing inert additives and, as active ingredient, a dibenzofuran derivative of the formula I as claimed in claim 1, where $R^1$ is halogen, alkyl or alkoxy of 1 to 4 carbon atoms, trifluoromethyl or nitro, n is 1, 2 or 3, p and q are O, X is oxygen, m is 2, 3 or 4, A is $-N^{\oplus}R^4R^5R^6$, $R^4$, $R^5$ and $R^6$ being identical or different and each independently of one another being unsubstituted or halogen-substituted alkyl of 1 to 5 carbon atoms or cycloalkyl of 3 to 6 carbon atoms, and $Y^{\ominus}$ is the anion of any desired non-phytotoxic acid HY.

7. A fungicidal agent containing inert additives and, as active ingredient, a dibenzofuran derivative of the formula I as claimed in claim 1, where $R^1$ is halogen, alkyl or alkoxy of 1 to 4 carbon atoms, trifluoromethyl or nitro, n is 1, p and q are 0, X is oxygen, m is 2,3 or 4, A is $-N^{\oplus}R^4R^5R^6$, $R^4$ being unsubstituted benzyl or benzyl substituted by fluorine, chlorine, bromine, triflurometyl, nitro or by alkyl of 1 to 4 carbon atoms, and $R^5$ together with $R^6$ being part of a pyrrolidinium, piperidinium, hexamethyleneiminium, morpholinium or thiomorpholinium ring which may be substituted by methyl or ethyl, and $Y^{\ominus}$ is the anion of any desired non-phytotoxic acid HY.

8. A process for combating fungi, wherein a dibenzofuran derivative of the formula I as claimed in claim 1 is allowed to act on the fungi, or on areas, plants or seed threatened by fungus attack.

**Revendications**

1. Dérivés du dibenzo-furanne de la formule

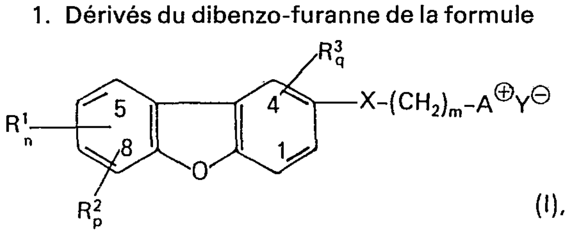

(I),

dans laquelle

$R^1$, $R^2$ et $R^3$, qui peuvent être identiques ou différents, désignent chacun un atome d'halogène, un radical alkyle ou alcoxy en $C_1$ à $C_4$ éventuellement halo-substitué ou un groupe cyano ou nitro,

n, p et q valent 0, 1, 2 ou 3,

X désigne un atome d'oxygène ou de soufre,

m vaut 2, 3, ou 4 et

A représente un groupe bicyclique quinuclidinique ou pyrrolizidinique ou un groupe $-N^{\oplus}R^4R^5R^6$, dans lequel $R^4$, $R^5$ et $R^6$, qui peuvent être identiques ou différents, désignent indépendamment l'un de l'autre, des groupes alkyle comportant jusqu'à 6 atomes de carbone, allyle, butényle-2, chloro-4 buténylе-2, propargyle, des groupes cycloalkyle comportant jusqu'à 12 atomes de carbone ou des groupes cycloalcényle comportant jusqu'à 7 atomes de carbone, ces groupes acycliques et cycliques pouvant être substitués par des atomes d'halogène ou des groupes cyano, alcoxy en $C_1$ à $C_4$ ou dialkyl-amino à radicaux alkyle en $C_1$ à $C_4$, ou des groupes aralkyle éventuellement substitués par des atomes de fluor, de chlore ou de brome, des radicaux alkyle, alcényle ou alcoxy comportant jusqu'a 4 atomes de carbone ou des groupes trifluorométhyle, nitro, cyano ou alcoxy-carbonyle comportant jusqu'à 5 atomes de carbone,

$R^5$ et $R^6$ pouvant aussi faire partie d'un reste pyrrolidinium, pipéridinium, hexaméthylèneiminium, morpholinium ou thiomorpholinium éventuellement méthyl- ou éthyl-substitué et

Y représente l'anion d'un acide HY non phytotoxique quelconque.

2. Dérivés du dibenzo-furanne de la formule I selon la revendication 1, caractérisés en ce que

$R^1$ désigne un atome d'halogène ou un radical alkyle ou alcoxy en $C_1$ à $C_4$ ou un groupe trifluorométhyle ou nitro,

n vaut 1, 2 ou 3,

$p = q = 0$,

X = oxygène,

m vaut 2, 3 ou 4,

$A = -N^{\oplus}R^4R^5R^6$, où $R^4$, $R^5$ et $R^6$ sont identiques ou différents et désignent indépendamment l'un de l'autre des radicaux alkyle en $C_1$ à $C_5$ éventuellement halo-substitués ou des groupes cycloalkyle en $C_3$ à $C_6$ et

Y désigne l'anion d'un acide HY non phytotoxique quelconque.

3. Dérivés du dibenzo-furanne de la formule I selon la revendication 1, caractérisés en ce que

$R^1$ désigne un atome d'halogène ou un radical alkyle ou alcoxy en $C_1$ à $C_4$ ou un groupe trifluorométhyle ou nitro,

n vaut 1,

$p = q = 0$,

X = oxygène,

m vaut 2, 3 ou 4,

$A = -N^{\oplus}R^4R^5R^6$, où $R^4$ désigne un groupe benzyle éventuellement substitué par des atomes de fluor, de chlore ou de brome, des groupes trifluoro-méthyle ou nitro ou alkyle en $C_1$ à $C_4$ et $R^5$ et $R^6$ font partie d'un reste pyrrolidinium, pipéridinium, hexaméthylèneiminium, morpholinium ou thiomorpholinium éventuellement méthyl- ou éthyl-substitué et
Y désigne l'anion d'un acide HY non phytotoxique quelconque.

4. Procédé de préparation de dérivés du dibenzo-furanne de la formule I selon la revendication 1, caractérisé en ce que l'on fait réagir:

a) des composés de la formule

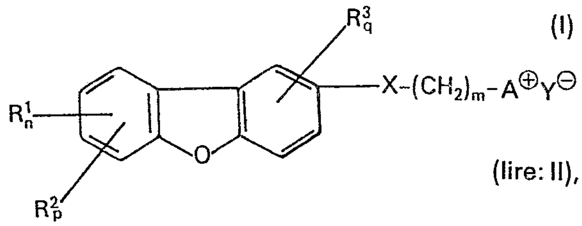

(I)

(lire: II),

dans laquelle $R^1$, $R^2$, $R^3$, X, Y, m, n, p, et q possèdent les significations définies dans la revendication 1, avec une amine tertiaire de la formule A telle que définie dans la revendication 1, ou

b) des composés de la formule

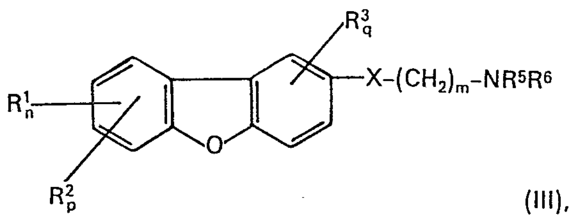

(III),

dans laquelle $R^1$, $R^2$, $R^3$, $R^5$, $R^6$, X, m, n, p et q possèdent les significations définies dans la revendication 1, avec un composé de la formule

$R^4$–Y (IV),

dans laquelle $R^4$ et Y possèdent les significations définies dans la revendication 1.

5. Composition fongicide, contenant des additifs inertes et en tant que principe actif un dérivé du dibenzo-furanne de la formule

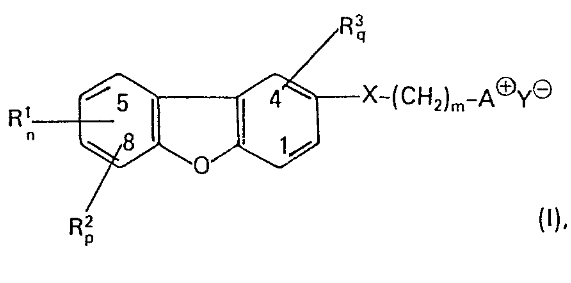

(I),

dans laquelle
$R^1$, $R^2$ et $R^3$, qui peuvent être identiques ou différents, désignent chacun un atome d'halogène, un radical alkyle ou alcoxy en $C_1$ à $C_4$ éventuellement halo-substitué ou un groupe cyano ou nitro,
n, p et q valent 0, 1, 2 ou 3,
X désigne un atome d'oxygène ou de soufre,
m vaut 2, 3 ou 4 et
A représente un groupe bicyclique quinuclidinique ou pyrrolizidinique ou un groupe $-N^{\oplus}R^4R^5R^6$, dans lequel $R^4$, $R^5$ et $R^6$, qui peuvent être identiques ou différents, désignent indépendamment l'un de l'autre, des groupes alkyle comportant jusqu'à 6 atomes de carbone, allyle, buténe-2, chloro-4 butényle-2, propargyle, des groupes cycloalkyle comportant jusqu'à 12 atomes de carbone ou des groupes cycloalcényle comportant jusqu'à 7 atomes de carbone, ces groupes acycliques et cycliques pouvant être substitués par des atomes d'halogène ou des groupes cyano, alcoxy en $C_1$ à $C_4$ ou dialkyl-amino à radicaux alkyle en $C_1$ à $C_4$, ou des groupes aralkyle éventuellement substitués par des atomes de fluor, de chlore ou de brome, des radicaux alkyle, alcényle ou alcoxy comportant jusqu'à 4 atomes de carbone ou des groupes trifluorométhyle, nitro, cyano ou alcoxy-carbonyle comportant jusqu'à 5 atomes de carbone,
$R^5$ et $R^6$ pouvant aussi faire partie d'un reste pyrrolidinium, pipéridinium, hexaméthylèneiminium, morpholinium ou thiomorpholinium éventuellement méthyl- ou éthyl-substitué et
Y représente l'anion d'un acide HY non phytotoxique quelconque.

6. Composition fongicide, contenant des additifs inertes et en tant que principe actif un dérivé du dibenzofuranne de la formule I selon la revendicaton, 1, dans laquelle
$R^1$ désigne un atome d'halogène ou un radical alkyle ou alcocy en $C_1$ à $C_4$ ou un groupe trifluoromethyle ou nitro,
n vaut 1, 2 ou 3,
p = q = 0,
X = oxygène,
m vaut 2, 3 ou 4,
A = $-N^{\oplus}R^4R^5R^6$, où $R^4$, $R^5$ et $R^6$ sont identiques ou différents et désignent indépendamment l'un de l'autre des radicaux alkyle en $C_1$ à $C_5$ éventuellement halosubstitués ou des groupes cycloalkyle en $C_3$ à $C_6$ et
Y désigne l'anion d'un acide NY non phytotoxique quelconque.

7. Composition fongicide, contenant des additifs inertes et en tant que principe actif un dérivé du dibenzofuranne de la formule I selon la revendication 1, dans laquelle
$R^1$ désigne un atome d'halogène ou un radical alkyle ou alcoxy en $C_1$ à $C_4$ ou un groupe trifluorométhyle ou nitro,
n vaut 1,
p = q = 0,
X = oxygène,
m vaut 2, 3 ou 4,
A = $-N^{\oplus}R^4R^5R^6$, où $R^4$ désigne un groupe benzyle éventuellement substitué par des atomes de fluor, de chlore ou de brome, des groupes trifluoro-méthyle ou nitro ou alkyle en $C_1$ à $C_4$ et $R^5$ et $R^6$ font partie d'un reste pyrrolidinium, pipéridinium, hexaméthylèneiminium, morpholinium ou thiomorpholinium éventuellement méthyl- ou éthyl-substitué et
Y désigne l'anion d'un acide HY non phytotoxique quelconque.

8. Procédé de lutte contre les champignons, caractérisé en ce que l'on laisse agir un dérivé du dibenzo-furanne de la formule I selon la revendication 1 sur les champignons ou sur les surfaces, plantes ou semences exposées aux champignons.